# EUROPEAN PATENT APPLICATION

(11) **EP 4 763 848 A1**
(43) Date of publication of application: **24.06.2026**
(21) Application number: 24861868.8
(22) Date of filing: 27.08.2024
(51) Int. Cl.: C07D 495/04, C09K 19/34

(54) **COMPOUND, LIQUID CRYSTAL COMPOSITION AND USES OF BOTH**

(30) Priority: 06.09.2023 CN 202311148441
(71) Applicant: HUAWEI TECHNOLOGIES CO., LTD., Shenzhen 518129 (CN)
(72) Inventor: CHEN, Quan, Shenzhen, Guangdong 518129 (CN); SI, Xiaojia, Shenzhen, Guangdong 518129 (CN); CHEN, Pingan, Shenzhen, Guangdong 518129 (CN); GONG, Lifeng, Shenzhen, Guangdong 518129 (CN)
(74) Representative: Körber, Martin Hans
(86) International application number: PCT/CN2024/114803
(87) International publication number: WO 2025/051024

(57) **Abstract**

Embodiments of this application relate to the field of liquid crystal technologies, and disclose a compound, a liquid crystal composition and uses thereof, to solve the technical problem of a short service life of an electronic device caused by poor material performance and a high material performance degradation speed of liquid crystal materials. The compound has a structure as set forth in formula (I).

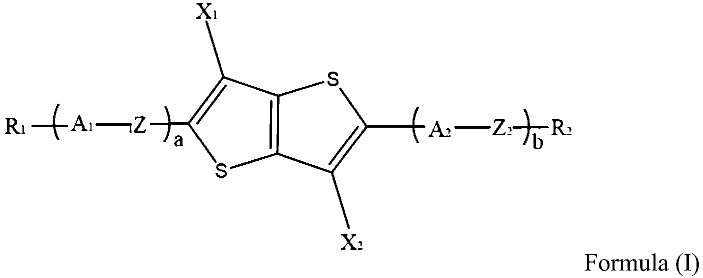

X₁ and X₂ are each independently selected from hydrogen, deuterium, tritium, at least one halogen and at least one hydrocarbyl group, and at least one of X₁ and X₂ is not hydrogen. The compound has a relatively high birefringence, relatively good dielectric anisotropy, a relatively low rotational viscosity, a relatively high clearing point, relatively good low-temperature stability and the like, and when the compound is applied as a liquid crystal compound, the performance of the liquid crystal material degrades at a low speed, so that the service life of the electronic device can be prolonged.

## Description

This application claims priority to Chinese Patent Application No. 202311148441.1, filed with the China National Intellectual Property Administration on September 6, 2023 and entitled "COMPOUND, LIQUID CRYSTAL COMPOSITION AND USES THEREOF", which is incorporated herein by reference in its entirety.

### TECHNICAL FIELD

This application relates to the field of liquid crystal technologies, and in particular, to a compound, a liquid crystal composition, and uses of the compound and the liquid crystal composition.

### BACKGROUND

Liquid crystal materials have been widely used in the fields such as smart phones, tablet equipment, televisions, and vehicle instruments. Further, there is also a wide demand for liquid crystal materials in the fields such as 3D display, optical communication, microwave antennas, and lenses. In these application scenarios, liquid crystal materials are required to support a larger phase and dielectric modulation amount, a faster switching speed, and a higher resolution.

The relationship between the phase modulation amount of a liquid crystal device and the birefringence and box thickness of a liquid crystal material used by the liquid crystal device is as follows: When the box thickness is fixed, the larger the birefringence of the liquid crystal material, the larger the phase modulation amount of the liquid crystal device. Response time of the liquid crystal device is directly proportional to a rotational viscosity of the liquid crystal material used by the liquid crystal device, and is inversely proportional to an elastic coefficient of the liquid crystal material used by the liquid crystal device. Therefore, in these application scenarios, the birefringence of the liquid crystal material is required to be obviously higher than that of a common liquid crystal, and at the same time, sufficient dielectric anisotropy, a relatively lower rotational viscosity, a relatively higher clearing point, low-temperature stability (less likely to crystallize at a low temperature), voltage holding ratio stability (less likely to drop), and the like also need to be considered.

Although liquid crystal materials provided in some related technologies have advantages such as a high birefringence, the material performance of the liquid crystal materials is likely to degrade. Therefore, providing a liquid crystal material giving consideration to a high birefringence and material performance stability becomes an urgent technical problem to be solved.

### SUMMARY

Embodiments of this application provide a compound, a liquid crystal composition, and uses of the compound and the liquid crystal composition in a display device, an optical communication device, a wireless communication device, a lens component, and the like, to solve the technical problem of a short service life of a device or a component caused by a low birefringence and a high material performance degradation speed of liquid crystal materials.

To achieve the aforementioned objective, embodiments of this application use the following technical solutions:
In a first aspect, a compound is provided, and the compound has a structure as set forth in formula (I),

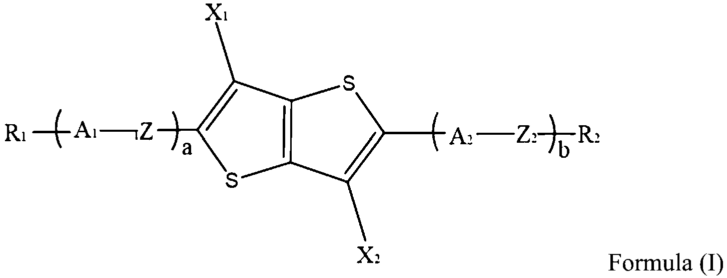

X₁ and X₂ are each independently selected from hydrogen, deuterium, tritium, at least one halogen and at least one hydrocarbyl group, and at least one of X₁ and X₂ is not hydrogen; R₁ is selected from at least one hydrocarbyl group; R₂ is selected from at least one polar group and at least one hydrocarbyl group; A₁ and A₂ are each independently selected from an arylene group, a heteroarylene group, a cyclic hydrocarbylene group, and a heterocyclic hydrocarbylene group; Z₁ is a linking group or a chemical bond; Z₂ is a linking group or a chemical bond; and a and b are integers.

The compound having the structure as set forth in formula (I) has a relatively high birefringence, relatively good dielectric anisotropy, a relatively low rotational viscosity, a relatively high clearing point, and relatively good low-temperature stability, and thus may satisfy the requirements, such as a larger phase and dielectric modulation amount, a higher switching speed, and a higher resolution, of an electronic device in the fields such as smart terminals, vehicle instruments, 3D display, optical communication, microwave antennas, and lenses (variable focus) for a liquid crystal material. In addition, because at least one hydrogen on a lateral side of a thienothiophene structure in the compound structure is substituted, and a substituent group is selected from deuterium, tritium, at least one halogen and at least one hydrocarbyl group, and the presence of the substituent group may improve the structural stability of the compound and reduce the reaction activity of the compound. Therefore, when used as a liquid crystal compound, the compound may reduce the performance degradation speed of a liquid crystal material, thereby prolonging the service life of an electronic device (various types of liquid crystal components or devices including the liquid crystal components).

With reference to the first aspect, in a possible implementation of the first aspect, when X₁ is selected from at least one hydrocarbyl group, X₁ is of a straight chain structure, or has a branched chain structure.

With reference to the first aspect, in a possible implementation of the first aspect, when X₂ is selected from at least one hydrocarbyl group, X₂ is of a straight chain structure, or has a branched chain structure.

With reference to the first aspect, in a possible implementation of the first aspect, when X₁ is selected from at least one hydrocarbyl group, at least one hydrogen atom on X₁ is substituted with a fluorine atom.

With reference to the first aspect, in a possible implementation of the first aspect, when X₂ is selected from at least one hydrocarbyl group, at least one hydrogen atom on X₂ is substituted with a fluorine atom.

With reference to the first aspect, in a possible implementation of the first aspect, when X₁ is selected from at least one hydrocarbyl group, X₁ includes 1 to 8 carbon atoms.

With reference to the first aspect, in a possible implementation of the first aspect, when X₂ is selected from at least one hydrocarbyl group, X₂ includes 1 to 8 carbon atoms.

With reference to the first aspect, in a possible implementation of the first aspect, X₁ and X₂ are each independently an alkyl group, an alkoxy group, an alkenyl group, an alkenyloxy group, a cycloalkyl group, or a cycloalkenyl group.

With reference to the first aspect, in a possible implementation of the first aspect, the alkyl group includes 1 to 8 carbon atoms; the alkoxy group includes 1 to 7 carbon atoms; the alkenyl group includes 2 to 8 carbon atoms; the alkenyloxy group includes 2 to 7 carbon atoms; the cycloalkyl group includes 3 to 8 carbon atoms; and the cycloalkenyl group includes 3 to 8 carbon atoms.

With reference to the first aspect, in a possible implementation of the first aspect, at least one of X₁ and X₂ is a methyl group.

With reference to the first aspect, in a possible implementation of the first aspect, R₁ and R₂ are each independently selected from an alkyl group, an alkoxy group, an alkenyl group, an alkenyloxy group, an alkynyl group and an alkynyloxy group that are of a straight chain structure or have a branched chain structure.

With reference to the first aspect, in a possible implementation of the first aspect, R₁ and R₂ each independently contains one or more of oxygen, sulfur and nitrogen.

With reference to the first aspect, in a possible implementation of the first aspect, any hydrogen atom on R₁ and/or R₂ is optionally substituted with a deuterium atom, a tritium atom or a halogen atom.

With reference to the first aspect, in a possible implementation of the first aspect, any two non-adjacent carbon atoms in R₁ and/or R₂ bond together to form a cyclic ring structure.

With reference to the first aspect, in a possible implementation of the first aspect, R₁ and R₂ are each independently selected from an alkyl group and an alkoxy group with 1 to 20 carbon atoms, and an alkenyl group, an alkenyloxy group, an alkynyl group and an alkynyloxy group with 2 to 20 carbon atoms.

With reference to the first aspect, in a possible implementation of the first aspect, the at least one polar group includes F, Cl, Br, CN, NO₂, SCN, NCS, NCSe, OCF₃, and SF₅.

With reference to the first aspect, in a possible implementation of the first aspect, at least one hydrogen atom on A₁ and/or A₂ is substituted with X₃, and X₃ is selected from a deuterium atom, a tritium atom, a halogen atom, and at least one hydrocarbyl group.

With reference to the first aspect, in a possible implementation of the first aspect, when X₃ is selected from at least one hydrocarbyl group, any hydrogen atom on X₃ is substituted with a fluorine atom.

With reference to the first aspect, in a possible implementation of the first aspect, when X₃ is selected from at least one hydrocarbyl group, X₃ is selected from an alkyl group, an alkoxy group, an alkenyl group and an alkenyloxy group of a straight chain or a branched chain structure, a cycloalkyl group, and a cycloalkenyl group.

With reference to the first aspect, in a possible implementation of the first aspect, when X₃ is selected from at least one hydrocarbyl group, X₃ is selected from a straight-chain or branched-chain alkyl group with 1 to 8 carbon atoms, a straight-chain or branched-chain alkoxy group with 1 to 7 carbon atoms, a straight-chain or branched-chain alkenyl group with 2 to 8 carbon atoms, a straight-chain or branched-chain alkenyloxy group with 2 to 7 carbon atoms, a cycloalkyl group with 3 to 8 carbon atoms, and a cycloalkenyl group with 3 to 8 carbon atoms. Optionally, any hydrogen atom on the alkyl group, the alkoxy group, the alkenyl group, the alkenyloxy group and the cycloalkenyl group is substituted with a fluorine atom.

With reference to the first aspect, in a possible implementation of the first aspect, the arylene group includes 6 to 60 carbon atoms; the heteroarylene group includes 2 to 60 carbon atoms; the cyclic hydrocarbylene group includes 3 to 60 carbon atoms; and the heterocyclic hydrocarbylene group includes 1 to 60 carbon atoms.

With reference to the first aspect, in a possible implementation of the first aspect, when a plurality of A₁s are present in formula (I), the plurality of A₁s are the same or different; and when a plurality of A₂s are present in formula (I), the plurality of A₂s are the same or different.

With reference to the first aspect, in a possible implementation of the first aspect, Z₁ and Z₂ are each independently selected from -CH=CH-, -CF=CF-, -CH=CF-, -CF=CH-, -C=C-, -C≡C-C≡C-, -CO-O-, -O-CO, -CF₂O-, -OCF₂-, -CH₂CH₂-, -N=N-, -CH=N-, -N=CH-, -CH₂O-, -OCH₂-, -CF₂CF₂-, -CF₂CH₂-, and -CH₂CF₂-.

With reference to the first aspect, in a possible implementation of the first aspect, a is selected from 0, 1, 2 and 3; and b is selected from 0, 1, 2 and 3.

In a second aspect, a liquid crystal composition is provided. The liquid crystal composition includes any one of the aforementioned compounds.

In a third aspect, uses of any one of the compounds according to the first aspect or the liquid crystal composition according to the second aspect in a display device, an optical communication device, a wireless communication device, and a lens component are provided.

For the technical effects brought in by any design manner in the second aspect and the third aspect, refer to the technical effects brought in by different design manners in the first aspect, and details thereof will not be repeated herein.

### BRIEF DESCRIPTION OF DRAWINGS

FIG. 1 is a diagram of a structure of an optical communication network according to some embodiments;
FIG. 2 is a diagram of a structure of a wavelength selective switch according to some embodiments; and
FIG. 3 is a diagram of a structure of an optical path modulation system according to some embodiments.

### DESCRIPTION OF EMBODIMENTS

The following describes the technical solutions in embodiments of this application with reference to the accompanying drawings in embodiments of this application. In descriptions of this application, unless otherwise specified, "/" represents an "or" relationship between associated objects. For example, A/B may represent A or B. In this application, "and/or" describes only an association relationship for describing associated objects and represents that three relationships may exist. For example, A and/or B may represent the following three cases: only A exists, both A and B exist, and only B exists, where A and B may be singular or plural.

In addition, in the descriptions of this application, "a plurality of" means two or more than two unless otherwise specified. "At least one of the following items" or a similar expression thereof means any combination of these items, including a singular item or any combination of a plurality of items. For example, at least one of a, b, or c may indicate: a, b, c, a and b, a and c, b and c, or a, b and c, where a, b, and c may be singular or plural.

In addition, in embodiments of this application, terms such as "exemplary" or "for example" are used for indicating an example, an illustration, or an explanation. Any embodiment or design scheme described as an "example" or "for example" in embodiments of this application should not be explained as being more preferred or having more advantages than other embodiments or design schemes. Exactly, use of the terms such as "exemplary" or "for example" is intended to present a related concept in a specific manner for ease of understanding.

In addition, a liquid crystal component and an application scenario described in embodiments of this application are intended to explain the technical solutions in embodiments of this application more clearly, and do not constitute a limitation on the technical solutions provided in embodiments of this application. A person of ordinary skill in the art may know that with the evolution of liquid crystal components and emergence of new application scenarios, the technical solutions provided in embodiments of this application are also applicable to similar technical problems.

Before specific implementations of the technical solutions of this application are described, terms involved in embodiments of this application are first described.

Hydrocarbyl group: From the perspective of atomic composition, hydrocarbyl groups may include a hydrocarbyl group formed by carbon and hydrogen; a hydrocarbyl group formed by carbon, hydrogen and oxygen; and a hydrocarbyl group containing heteroatoms formed by carbon, hydrogen and heteroatoms, where the heteroatoms may be oxygen, sulfur and nitrogen. In addition, hydrocarbyl groups may also include a substituted hydrocarbyl group in which any one or more hydrogen atoms are substituted with a halogen atom or the like. From the perspective of group structure, a hydrocarbyl group may be of a chain structure, or may be of a cyclic structure. The chain structure may include a straight chain structure or a branched chain structure (also referred to as a branched chain). From the perspective of saturation, hydrocarbyl groups may include saturated hydrocarbyl groups and unsaturated hydrocarbyl groups.

Polar group: An organic moiety of a molecule in which electron distribution produce significant electrical dipole moments. Such a group presents affinity to low-polar organic solvents and determines the oleophilic property of the molecule.

As described in the background, in more and more application scenarios, a liquid crystal material is required to have a relatively high birefringence, and at the same time, sufficient dielectric anisotropy, a relatively low rotational viscosity, a relatively high clearing point, low-temperature stability (less likely to crystallize at a low temperature), voltage holding ratio stability (less likely to drop), and the like also need to be considered. However, the material performance is likely to degrade, and the stability of the performance cannot be guaranteed.

In view of this, embodiments of this application provide a compound, a liquid crystal composition (also referred to as a liquid crystal material) including the compound, and an electronic device including the liquid crystal material. The liquid crystal material has material performance such as a relatively high birefringence (Δn), relatively good dielectric anisotropy (Δε), a relatively low rotational viscosity (γ₁), a relatively high clearing point, low-temperature stability (less likely to crystallize at a low temperature), and voltage holding ratio stability, and also has relatively high material performance stability.

The compound or the liquid crystal composition including the compound provided by embodiments of this application may be applied to application scenarios such as optical communication, wireless communication, microwave scanning antennas, lenses (for example, a variable-focus lens), optical grating, laser radars, beam tracking, projection, flat-panel display, and holographic display. The electronic device provided by embodiments of this application includes, but is not limited to, an electronic device in the aforementioned application scenarios.

For example, the electronic device may be user equipment or terminal devices of different types such as a mobile phone, a tablet computer, a personal digital assistant (Personal Digital Assistant, PDA for short), a laser television, a projector, a smart wearable product (for example, a smart watch or a smart band), a virtual reality (Virtual Reality, VR for short) terminal device, an augmented reality (Augmented Reality, AR for short) terminal device, a small rechargeable household appliance, a drone, radar, an aerospace device, and an on-board device. The electronic device may also be an optical communication device such as a wavelength selective switch (Wavelength Selective Switch, WSS for short), a wireless communication device, a microwave scanning antenna, a lens component, an optical grating element, a laser radar element, a beam tracker, a projector, a holographic polymer dispersed liquid crystal component, a flat-panel display, and a holographic display. The specific form of the electronic device is not specifically limited in embodiments of this application.

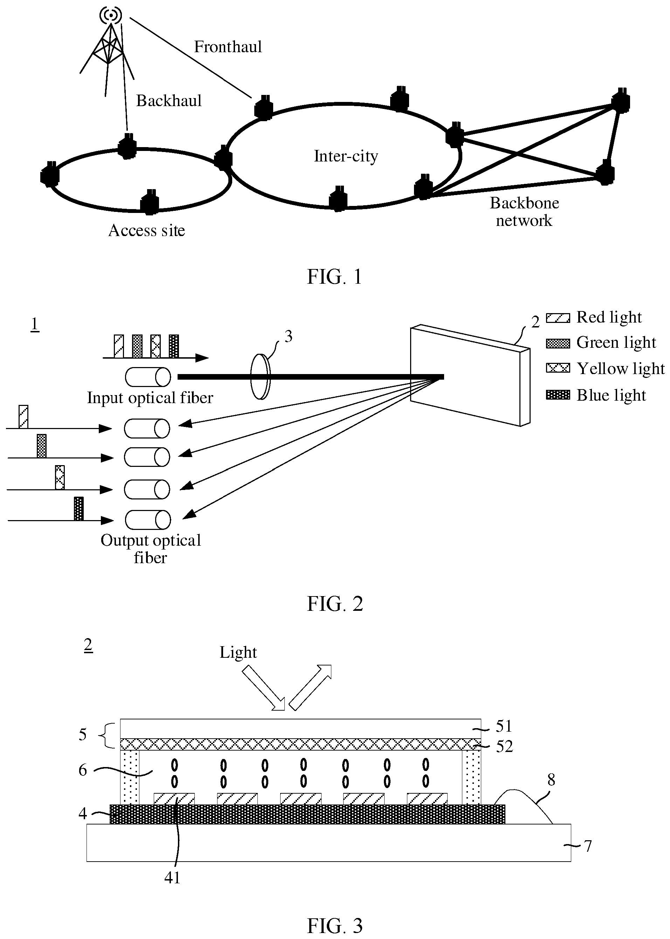

For example, the electronic device is a wavelength selective switch. FIG. 1 is a diagram of a structure of an optical communication network according to some embodiments, and FIG. 2 is a diagram of a structure of a wavelength selective switch according to some embodiments.

Referring to FIG. 1 and FIG. 2, a wavelength selective switch 1 may be widely used in key nodes in an optical communication network, for example, may be used in an inter-city network or a long-distance backbone network, and implement data uploading and downloading by scheduling a wavelength of an optical signal. Specifically, the wavelength selective switch 1 may independently schedule and control optical signals of different wavelengths in an input optical fiber (a common port), and randomly schedule the optical signals of different wavelengths to different output optical fibers (output ports).

The wavelength selective switch 1 includes an optical path modulation system 2 and a beam splitting lens 3 shown in FIG. 3. Referring to FIG. 3, the optical path modulation system 2 includes a liquid crystal on silicon chip 4, an opposite substrate 5, a liquid crystal layer 6, and a flexible printed circuit (Flexible Printed Circuit, FPC for short) 7. The liquid crystal on silicon chip 4 may be electrically connected to the flexible printed circuit 7 via a welding wire 8. The liquid crystal on silicon chip 4 is arranged on the flexible printed circuit 7, and the liquid crystal on silicon chip 4 includes a pixel electrode 41 located on an upper surface of the liquid crystal on silicon chip 4. The opposite substrate 5 and the liquid crystal on silicon chip 4 are arranged opposite to each other. The opposite substrate 5 includes a common electrode 52, and a driving electric field may be formed between the common electrode 52 and the pixel electrode 41. The opposite substrate 5 further includes a substrate 51. The common electrode 52 is arranged on the substrate 51 and is located on one side of the substrate 51 close to the liquid crystal on silicon chip 4. The liquid crystal layer 6 is arranged between the liquid crystal on silicon chip 4 and the opposite substrate 5. Liquid crystal molecules in the liquid crystal layer 6 may rotate under the driving of the driving electric field, to adjust the reflectivity of the liquid crystal molecules to light of different wavelengths. Therefore, light of different wavelengths in the input optical fiber may be reflected at different emergent angles, and selective light output is achieved, that is, independent scheduling and control of an optical signal are achieved.

The liquid crystal layer 6 includes a liquid crystal composition. The liquid crystal composition may include a compound A. A main chain structure of the compound A includes a thienothiophene structure, and at least one hydrogen atom on a lateral side of the thienothiophene structure is substituted with X, where X may be selected from deuterium, tritium, at least one halogen, and at least one hydrocarbyl group.

In some embodiments, the compound A has a structure as set forth in the following formula (I):

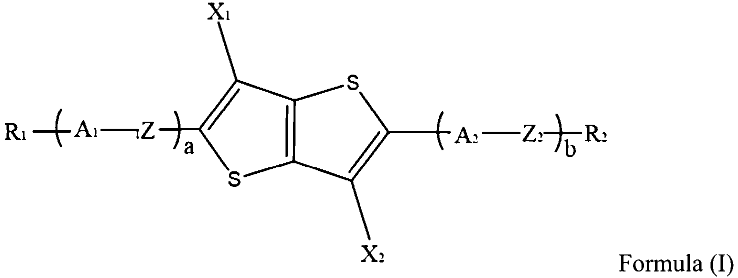

In formula (I), R₁ is selected from at least one hydrocarbyl group; R₂ is selected from at least one polar group and at least one hydrocarbyl group; A₁ and A₂ are each independently selected from an arylene group, a heteroarylene group, a cyclic hydrocarbylene group, and a heterocyclic hydrocarbylene group; Z₁ is a linking group or a chemical bond for linking A₁ with the thienothiophene structure; Z₂ is a linking group or a chemical bond for linking A₂ with the thienothiophene structure; and a and b are integers.

As can be seen from formula (I), the main chain of the compound is formed by the thienothiophene structure, A₁, A₂, R₁, R₂, Z₁, and Z₂. X₁ and X₂ are present on the lateral side of the thienothiophene structure, and X₁ and X₂ are each independently selected from hydrogen, deuterium, tritium, at least one halogen, and at least one hydrocarbyl group. At least one of X₁ and X₂ is not hydrogen. That is, when X₁ is hydrogen, X₂ is not hydrogen, and a hydrogen atom on the lateral side of the thienothiophene structure is substituted with X₂. When X₂ is hydrogen, X₁ is not hydrogen, and a hydrogen atom on the thienothiophene structure is substituted with X₁. When neither X₁ nor X₂ is hydrogen, the two hydrogen atoms on the lateral side of the thienothiophene structure are substituted with X₁ and X₂ respectively.

The compound having the structure as set forth in formula (I) has a relatively high birefringence, relatively good dielectric anisotropy, a relatively low rotational viscosity, a relatively high clearing point, and relatively good low-temperature stability, and thus may satisfy the requirements, such as a larger phase and dielectric modulation amount, a higher switching speed, and a higher resolution, in the fields such as smart terminals, vehicle instruments, 3D display, optical communication, microwave antennas, and lenses for a liquid crystal material. In addition, because at least one hydrogen on the lateral side of the thienothiophene structure in the compound structure is substituted, and a substituent group is selected from deuterium, tritium, at least one halogen and at least one hydrocarbyl group, and the presence of the substituent group may improve the structural stability of the compound and reduce the reaction activity of the compound. Therefore, when used as a liquid crystal compound, the compound may reduce the performance degradation speed of a liquid crystal material, thereby prolonging the service life of an electronic device (various types of liquid crystal components or devices including liquid crystal components).

In some embodiments, when X₁ and X₂ are each independently selected from at least one hydrocarbyl group, X₁ and X₂ are each independently of a straight chain structure, or has a branched chain structure. For ease of description, a hydrocarbyl group having a branched chain structure is referred to as a branched-chain hydrocarbyl group hereinafter. For example, X₁ and X₂ are each independently a straight-chain or branched-chain alkyl group, alkoxy group, alkenyl group, alkenyloxy group, cycloalkyl group, or cycloalkenyl group.

In some embodiments, when X₁ and X₂ are each independently selected from at least one hydrocarbyl group, at least one hydrogen atom on X₁ and/or X₂ is substituted with a fluorine atom. For example, X₁ and X₂ are each independently a straight-chain or branched-chain alkyl group, alkoxy group, alkenyl group, alkenyloxy group, cycloalkyl group or cycloalkenyl group. At least one hydrogen atom on the alkyl group, the alkoxy group, the alkenyl group, the alkenyloxy group, the cycloalkyl group and the cycloalkenyl group is substituted with a fluorine atom. In these embodiments, because at least one hydrogen atom on X₁ and/or X₂ is substituted with a fluorine atom, the dielectric property of the compound may be increased, the solubility of the compound may be improved, and the viscosity of the compound may be reduced. Therefore, when the compound is used for preparing a liquid crystal composition, the liquid crystal composition may have a larger phase and dielectric modulation amount, a higher switching speed, a higher resolution, and the like.

In some embodiments, when X₁ and X₂ are each independently selected from at least one hydrocarbyl group, X₁ and X₂ each independently includes 1 to 8 carbon atoms. For example, X₁ and X₂ are each independently: a straight-chain or branched-chain alkyl group with 1 to 8 carbon atoms, a straight-chain or branched-chain alkoxy group with 1 to 7 carbon atoms, a straight-chain or branched-chain alkenyl group with 2 to 8 carbon atoms, a straight-chain or branched-chain alkenyloxy group with 2 to 7 carbon atoms, a straight-chain or branched-chain cycloalkyl group with 3 to 8 carbon atoms, and a straight-chain or branched-chain cycloalkenyl group with 3 to 8 carbon atoms. In these embodiments, since X₁ and X₂ each independently include 1 to 8 carbon atoms, the viscosity and the solubility of the compound may be within preferred ranges.

In some embodiments, at least one of X₁ and X₂ is a methyl group. Specifically, when X₁ is a methyl group, X₂ may be selected from hydrogen, deuterium, tritium, at least one halogen, and at least one hydrocarbyl group as described in the foregoing embodiments. When X₂ is a methyl group, X₁ may be selected from hydrogen, deuterium, tritium, at least one halogen, and at least one hydrocarbyl group as described in the foregoing embodiments. Certainly, both X₁ and X₂ may be methyl groups. In these embodiments, because at least one of X₁ and X₂ is a methyl group, the low-temperature stability of the compound may be better. For example, in a relatively low temperature condition, the compound is less likely to crystallize, or rather, a critical temperature for crystallization is even lower.

It should be noted that the various implementations of X₁ and the various implementations of X₂ mentioned in the foregoing embodiments are merely examples and do not constitute a limitation on embodiments of this application. It should be noted that, in the structure of the compound A provided by embodiments of this application, X₁ and X₂ may be any one of hydrogen, deuterium, tritium, at least one halogen and at least one hydrocarbyl group, which will not be listed one by one herein.

In some embodiments, R₁ and R₂ are each independently of a straight chain structure or have a branched chain structure. For example, R₁ and R₂ are each independently selected from a straight-chain or branched-chain alkyl group, alkoxy group, alkenyl group, alkenyloxy group, alkynyl group and alkynyloxy group.

Optionally, R₁ and R₂ each independently contain heteroatoms, for example, contain one or more of oxygen, sulfur and nitrogen. For example, R₁ and R₂ are each independently selected from a straight-chain or branched-chain alkyl group, alkoxy group, alkenyl group, alkenyloxy group, alkynyl group and alkynyloxy group containing one or more of oxygen, sulfur and nitrogen.

Optionally, any hydrogen atom on R₁ and/or R₂ is optionally substituted with a deuterium atom, a tritium atom or a halogen atom. For example, R₁ and R₂ are each independently selected from a straight-chain or branched-chain alkyl group, alkoxy group, alkenyl group, alkenyloxy group, alkynyl group and alkynyloxy group of which any hydrogen atom is optionally substituted with a deuterium atom, a tritium atom or a halogen atom.

Optionally, any two non-adjacent carbon atoms in R₁ and/or R₂ bond together to form a cyclic structure. For example, R₁ and R₂ are each independently selected from a straight-chain or branched-chain alkyl group, alkoxy group, alkenyl group, alkenyloxy group, alkynyl group and alkynyloxy group, and any two non-adjacent carbon atoms in the alkyl group, the alkoxy group, the alkenyl group, the alkenyloxy group, the alkynyl group and the alkynyloxy group bond together to form a cyclic ring structure. The alkyl group and the alkoxy group may include 1 to 20 carbon atoms; and the alkenyl group, the alkenyloxy group, the alkynyl group and the alkynyloxy group may include 2 to 20 carbon atoms. In these embodiments, the cyclic structure may increase the degree of order of molecular packing, thereby improving the dielectric anisotropy of the compound. In addition, because the hydrocarbyl group from which R₁ and R₂ are selected includes 1 to 20 carbon atoms, the viscosity and the solubility of the compound may be within preferred ranges.

In some examples, R₁ and R₂ are each independently selected from a straight-chain or branched-chain alkyl group or alkoxy group with 1 to 20 carbon atoms and a straight-chain or branched-chain alkenyl group, alkenyloxy group, alkynyl group and alkynyloxy group with 2 to 20 carbon atoms. The alkyl group, the alkoxy group, the alkenyl group, the alkenyloxy group, the alkynyl group and the alkynyloxy group may optionally contain one or more heteroatoms of oxygen, sulfur, nitrogen and the like; any hydrogen atom on the alkyl group, the alkoxy group, the alkenyl group, the alkenyloxy group, the alkynyl group and the alkynyloxy group is optionally substituted with a deuterium atom, a tritium atom or a halogen atom; and any two non-adjacent carbon atoms in the alkyl group, the alkoxy group, the alkenyl group, the alkenyloxy group, the alkynyl group and the alkynyloxy group bond together to form a cyclic ring structure.

In a specific example, R₁ is selected from the following structures:

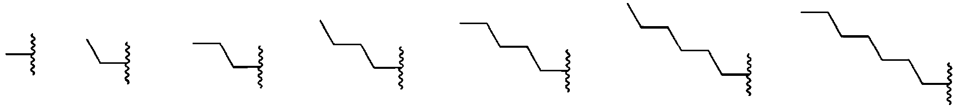

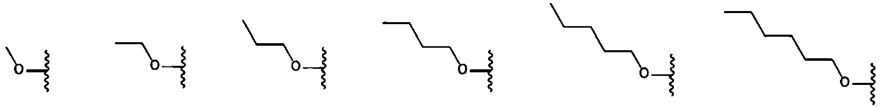

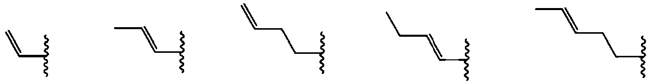

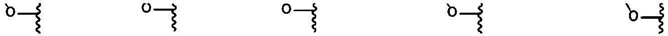

In some embodiments, the polar group from which R₂ is selected includes F, Cl, Br, CN, NO₂, SCN, NCS, NCSe, OCF₃, and SF₅.

It should be noted that the various implementations of R₁ and the various implementations of R₂ mentioned in the foregoing embodiments are merely examples and do not constitute a limitation on embodiments of this application. It should be noted that, in the structure of the compound A provided by embodiments of this application, R₁ may be any one of at least one hydrocarbyl group; and R₂ may be any one of at least one polar group and at least one hydrocarbyl group, which will not be listed one by one herein.

In some embodiments, A₁ and A₂ are each independently selected from an arylene group containing 6 to 60 carbon atoms, a heteroarylene group containing 2 to 60 carbon atoms, a cyclic hydrocarbylene group containing 3 to 60 carbon atoms, and a heterocyclic hydrocarbylene group containing 1 to 60 carbon atoms. In these embodiments, since the arylene group contains 6 to 60 carbon atoms, the heteroarylene group contains 2 to 60 carbon atoms, the cyclic hydrocarbylene group contains 3 to 60 carbon atoms, and the heterocyclic hydrocarbylene group contains 1 to 60 carbon atoms, the size of the cyclic structure may be ensured to be within a preferred range, thereby ensuring the low-temperature stability of the compound and making the compound less likely to crystallize in a low-temperature environment.

In some embodiments, at least one hydrogen atom on A₁ and/or A₂ is substituted with X₃. X₃ is selected from a deuterium atom, a tritium atom, a halogen atom and at least one hydrocarbyl group. If a plurality of hydrogen atoms on A₁ and A₂ are each independently substituted with X₃, a plurality of X₃s that substitute the plurality of hydrogen atoms may be the same or different. For example, A₁ and A₂ are each independently selected from an arylene group that contains 6 to 60 carbon atoms and on which at least one hydrogen atom is substituted with X₃, a heteroarylene group that contains 2 to 60 carbon atoms and on which at least one hydrogen atom is substituted with X, a cyclic hydrocarbylene group that contains 3 to 60 carbon atoms and on which at least one hydrogen atom is substituted with X₃, and a heterocyclic hydrocarbylene group that contains 1 to 60 carbon atoms and on which at least one hydrogen atom is substituted with X₃.

In some embodiments, when X₃ is selected from at least one hydrocarbyl group, X₃ may be specifically selected from a straight-chain or branched-chain alkyl group with 1 to 8 carbon atoms, a straight-chain or branched-chain alkoxy group with 1 to 7 carbon atoms, a straight-chain or branched-chain alkenyl group with 2 to 8 carbon atoms, a straight-chain or branched-chain alkenyloxy group with 2 to 7 carbon atoms, a cycloalkyl group with 3 to 8 carbon atoms, and a cycloalkenyl group with 3 to 8 carbon atoms. Optionally, any hydrogen atom on the alkyl group, the alkoxy group, the alkenyl group, the alkenyloxy group and the cycloalkenyl group is substituted with a fluorine atom.

In some embodiments, when a plurality of A₁s are present in formula (I), that is, when a is greater than 0, the plurality of A₁s are the same or different; and when a plurality of A₂s are present in formula (I), that is, when b is greater than 0, the plurality of A₂s are the same or different.

In some specific examples, A₁ and A₂ are each independently selected from 1,4-phenylene, 1,3-phenylene, 1,4-naphthylene, 2,6-naphthylene, 1,3-naphthylene, and 1,5-naphthylene; 2,5-furylene, 2,4-furylene, 2,5-thienylene, 2,4-thienylene, 2,5-selenophenylene, 2,4-selenophenylene, 2,5-benzofurylene, 2,6-benzofurylene, 2,5-benzothienylene, 2,6-benzothienylene, 2,5-benzoselenophenylene, 2,6-benzoselenophenylene, 3,7-dibenzofurylene, 2,6-dibenzofurylene, 2,7-dibenzofurylene, 3,7-dibenzothienylene, 2,6-dibenzothienylene, 2,7-dibenzothienylene, 3,7-dibenzoselenophenylene, 2,6-dibenzoselenophenylene, 2,7-dibenzoselenophenylene, 2,5-thieno[3,2-b]thienylene, 2,5-seleno[3,2-b]selenophenylene, 2,5-seleno[3,2-b]thienylene, 2,5-pyridylene, 2,5-pyrimidinylene, 2,5-pyrazinylene, 3,6-pyridazinylene, 2,6-quinolylene, 3,7-quinolylene, 4,8-quinolylene, 5,8-quinolylene, 1,4-isoquinolylene, 1,5-isoquinolylene, 3,7-isoquinolylene, 4,8-isoquinolylene, 5,8-isoquinolylene, 5,8-quinoxalylene; cis-1,4-cyclohexylene, trans-1,4-cyclohexylene, 1,4-cyclohexenylene, bicyclic[2.2.2]octane-1,4-diyl, 1,2,3,4-tetrahydronaphthalene-2,6-diyl, 5,6,7,8-tetrahydronaphthalene-1,4-diyl, decahydronaphthalene-2,6-diyl, and 2,3-dihydro-1H-indene-2,5-diyl. Optionally, one -CH₂- or two or more non-adjacent -CH₂- in the foregoing groups may be each independently substituted with -O- or -S-. A₁ and A₂ provided herein are merely examples, and do not constitute a limitation on embodiments of this application.

In some embodiments, Z₁ and Z₂ are each independently selected from -CH=CH-, - CF=CF-, -CH=CF-, -CF=CH-, -C=C-, -C=C-C=C-, -CO-O-, -O-CO, -CF₂O-, -OCF₂-, -CH₂CH₂-, -N=N-, -CH=N-, -N=CH-, -CH₂O-, -OCH₂-, -CF₂CF₂-, -CF₂CH₂-, and -CH₂CF₂-.

It should be noted that the various implementations of Z₁ and the various implementations of Z₂ mentioned in the foregoing embodiments are merely examples and do not constitute a limitation on embodiments of this application. It should be noted that Z₁ provided by embodiments of this application may be any linking group or chemical bond for linking A₁ with the thienothiophene structure, which will not be listed one by one herein. Z₂ may be any linking group or chemical bond for linking A₂ with the thienothiophene structure, which will not be listed one by one herein.

In some embodiments, a is selected from 0, 1, 2 and 3; and b is selected from 0, 1, 2 and 3. In these embodiments, because both a and b are less than or equal to 3, the main chain structure of the compound may be ensured to be within a preferred range. Therefore, the viscosity and the solubility of the compound are appropriate, and the low-temperature stability of the liquid crystal composition is guaranteed.

The following describes, by using specific embodiments, the compound and the liquid crystal composition including the compound provided by embodiments of this application, and the performance of the compound and the liquid crystal composition prepared in the specific embodiments is tested.

### Embodiment 1

### (1) Preparation of 2-bromo-3-methyl-5-(4-propylphenyl)thieno[3,2-b]thiophene, "A-1-C" for short.

Specifically, 5.2 g (14.50 mmol) of a raw material (referred to as "A-1-A"), 840 mg (0.7 mmol) of tetrakis(triphenylphosphine)palladium (Pd(PPh₃)₄ for short), and 6.14 g (29 mmol) of potassium phosphate (K₃PO₄ for short) were weighed and added to a three-necked flask (250 mL). The system was kept sealed, and the gas in the three-necked flask was replaced with argon. 13 mL of water and 52 mL of toluene were added for reaction. After the reaction temperature was increased to 75°C, 2.85 g (17.4 mmol) of a substance ("A-1-B" for short) dissolved in dimethyl sulfoxide (DMSO for short) was added slowly using an automatic sample injector for reaction for 3 hours. After the reaction was completed, the system was cooled to room temperature, and a product was sequentially filtered with silica gel, washed with water twice, and extracted with ethyl acetate to obtain an organic phase. The organic phase was sequentially washed with a saturated ammonium chloride aqueous solution and a saturated sodium chloride aqueous solution, twice respectively, and dried with anhydrous sodium sulfate. After filtration and spin drying, column chromatography was performed using n-hexane as an eluent for separation and purification to obtain 4.45 g of white solid "A-1-C", with a chromatographic purity (GC purity for short hereinafter) of 99.6%.

### (2) Preparation of 2-[(4-butyl-2,6-difluorophenyl)ethynyl]-3-methyl-5-(4-propylphenyl)thieno[3,2-b]thiophene, "A-1" for short.

Specifically, 4.45 g (12.67 mmol) of the substance "A-1-C", 278 mg (0.38 mmol) of [1,1'-bis(diphenylphosphino)ferrocene]dichloropalladium (Pd(dppf)Cl₂ for short), and 144 mg (0.76 mmol) of copper iodide (CuI for short) were weighed and added to a three-necked flask (250 mL). The system was kept sealed, and the gas in the three-necked flask was replaced with argon. 2.95 g (15.2 mmol) of a raw material "A-1-D", 20 mL of diisopropylamine, and 60 mL of DMSO were added for reaction in a sealed argon environment. After the reaction temperature was increased to 120°C, the reaction continued for 3 hours. After the reaction was completed, the system was cooled to room temperature, and a product was sequentially filtered with silica gel, washed with water twice, and extracted with ethyl acetate to obtain an organic phase. The organic phase was sequentially washed with a saturated ammonium chloride aqueous solution and a saturated sodium chloride aqueous solution, twice respectively, and dried with anhydrous sodium sulfate. After filtration and spin drying, column chromatography was performed using n-hexane as an eluent for separation and purification to obtain a faint yellow solid. The faint yellow solid was further triturated with methanol to obtain 4.45 g of a substance "A-1", with a GC purity of 99.98%, and gas chromatography-mass spectrometry (referred to as GC-MS hereinafter) (EI)m/z=464.

A reaction process as described in (1) and (2) in Embodiment 1 may be as set forth in the following formula:

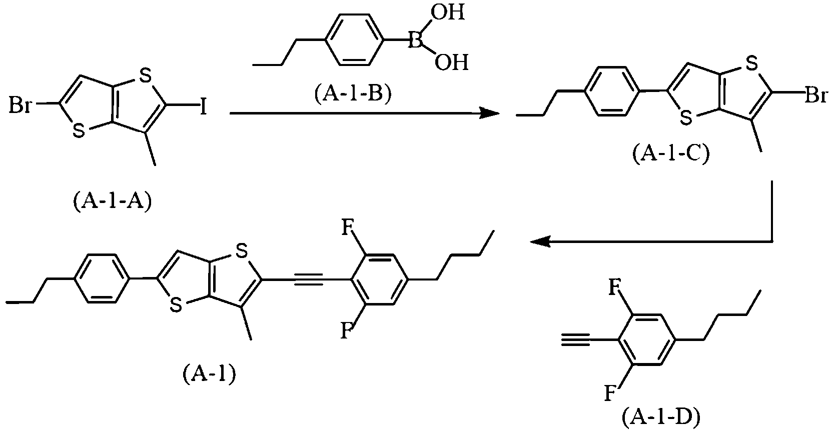

Based on the above, a structural formula of the compound A provided in Embodiment 1 of this application is:

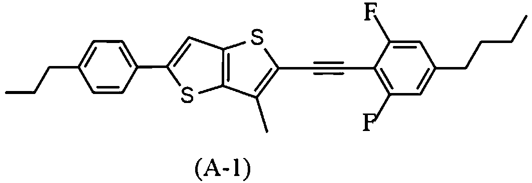

### Embodiment 2

### (1) Preparation of 3-methyl-2-{[4-(4-pentylphenyl)phenyl]ethynyl}thieno[3,2-b]thiophene, "A-2-C" for short.

Specifically, a raw material "A-2-B" (9.1 g, 36.9 mmol), [1,1'-bis(diphenylphosphino)ferrocene]dichloropalladium (Pd(dppf)Cl₂ for short) (808 mg, 1.1 mmol), and copper iodide (CuI) (421 mg, 2.2 mmol) were added to a three-necked flask (500 mL). The system was kept sealed, and the gas in the three-necked flask was replaced with argon. A raw material "A-2-A" (8.6 g, 36.9 mmol), diisopropylamine (20 mL), and DMSO (172 mL) were added. After the reaction temperature was increased to 120°C, the reaction continued for 3 hours. After the reaction was completed, the system was cooled to room temperature, and a product was sequentially filtered with silica gel, washed with water twice, and extracted with ethyl acetate to obtain an organic phase. The organic phase was sequentially washed with a saturated ammonium chloride aqueous solution and a saturated sodium chloride aqueous solution, twice respectively, and dried with anhydrous sodium sulfate. After filtration and spin drying, column chromatography was performed using n-hexane as an eluent for separation and purification to obtain a faint yellow solid. The faint yellow solid was further triturated with methanol to obtain 12.16 g of a substance "A-2-C", with a GC purity of 99.9%.

### (2) Preparation of 3-methyl-2-{[4-(4-pentylphenyl)phenyl]ethynyl}-5-propylthieno[3,2-b]thiophene, "A-2" for short.

Specifically, a raw material "A-2-C" (8 g, 20 mmol) was added to a three-necked flask (500 mL). The system was kept sealed, and the gas in the three-necked flask was replaced with argon. Tetrahydrofuran (THF for short) (240 mL) was added and stirred to dissolve until the solution is clear. The system was placed in a -78°C low-temperature reactor and stirred for 0.5 h. Lithium diisopropylamide (LDA for short) (18 mL, 36 mmol) was dropwise added for reaction for 3 h. A-2-D (6.11 g, 36 mmol) was dropwise added, and the temperature was increased to room temperature for reaction for 1 h. The reaction was quenched with a saturated ammonium chloride aqueous solution, and a product was sequentially washed with water twice and extracted with ethyl acetate to obtain an organic phase. The organic phase was sequentially washed with a saturated ammonium chloride aqueous solution and a saturated sodium chloride aqueous solution, twice respectively, and dried with anhydrous sodium sulfate. After filtration and spin drying, column chromatography was performed using n-hexane as an eluent for separation and purification to obtain a white solid. The white solid was further recrystallized and triturated with an ethyl acetate/methanol mixed solution for multiple times, to obtain 6.8 g of a substance "A-2", with a GC purity of 99.9%, and GC-MS (EI) m/z=442.

A reaction process as described in (1) and (2) in Embodiment 2 is as set forth in the following formula:

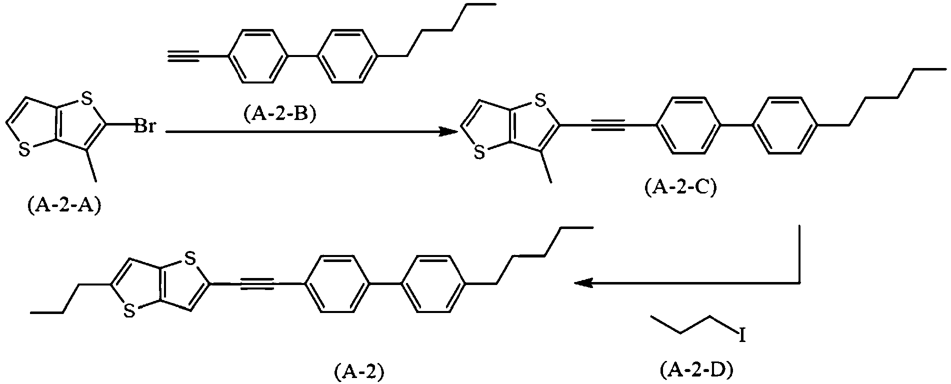

Based on the above, a structural formula of the compound A provided in Embodiment 2 of this application is:

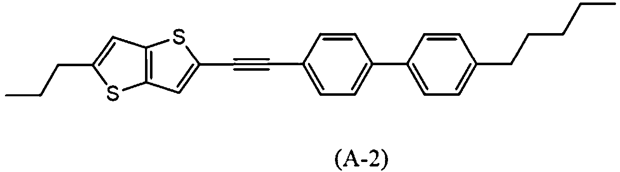

### Embodiment 3

### (1) Preparation of 2-{[4-(4-butylphenyl)phenyl]ethynyl}-3-methylthieno[3,2-b]thiophene, "A-3-C" for short.

Specifically, a raw material "A-3-B" (7.2 g, 31.1 mmol), [1,1'-bis(diphenylphosphino)ferrocene]dichloropalladium (Pd(dppf)Cl₂ for short) (452 mg, 0.6 mmol), and copper iodide (CuI for short) (235 mg, 1.2 mmol) were added to a three-necked flask (500 mL). The system was kept sealed, and the gas in the three-necked flask was replaced with argon. A raw material "A-3-A" (7.2 g, 31.1 mmol), diisopropylamine (18 mL), and DMSO (150 mL) were added. After the reaction temperature was increased to 120°C, the reaction continued for 3 hours. After the reaction was completed, the system was cooled to room temperature, and a product was sequentially filtered with silica gel, washed with water twice, and extracted with ethyl acetate to obtain an organic phase. The organic phase was sequentially washed with a saturated ammonium chloride aqueous solution and a saturated sodium chloride aqueous solution, twice respectively, and dried with anhydrous sodium sulfate. After filtration and spin drying, column chromatography was performed using n-hexane as an eluent for separation and purification to obtain a white solid. The white solid was further triturated with methanol to obtain 9.93 g of a substance "A-3-C", with a GC purity of 99.9%.

### (2) Preparation of 3-methyl-2-{[4-(4-pentylphenyl)phenyl]ethynyl}-5-propylthieno[3,2-b]thiophene, "A-3" for short.

Specifically, a raw material "A-3-C" (8 g, 20.7 mmol) was added to a three-necked flask (500 mL). The system was kept sealed, and the gas in the three-necked flask was replaced with argon. Tetrahydrofuran (THF for short) (160 mL) was added and stirred to dissolve until the solution is clear. The system was placed in a -78°C low-temperature reactor and stirred for 0.5 h. Lithium diisopropylamide (LDA for short) (21.7 mL, 43.4 mmol) was dropwise added for reaction for 3 h. "A-3-D" (7.4 g, 43.4 mmol) was dropwise added, and the temperature was increased to room temperature for reaction for 1 h. The reaction was quenched with a saturated ammonium chloride aqueous solution, and a product was sequentially washed with water twice and extracted with ethyl acetate to obtain an organic phase. The organic phase was sequentially washed with a saturated ammonium chloride aqueous solution and a saturated sodium chloride aqueous solution, twice respectively, and dried with anhydrous sodium sulfate. After filtration and spin drying, column chromatography was performed using n-hexane as an eluent for separation and purification to obtain a white solid. The white solid was further recrystallized and triturated with an ethyl acetate/methanol mixed solution for multiple times, to obtain 4.14 g of "A-3", with a GC purity of 99.9%, and GC-MS (EI) m/z=428.

A reaction process as described in (1) and (2) in Embodiment 3 is as set forth in the following formula:

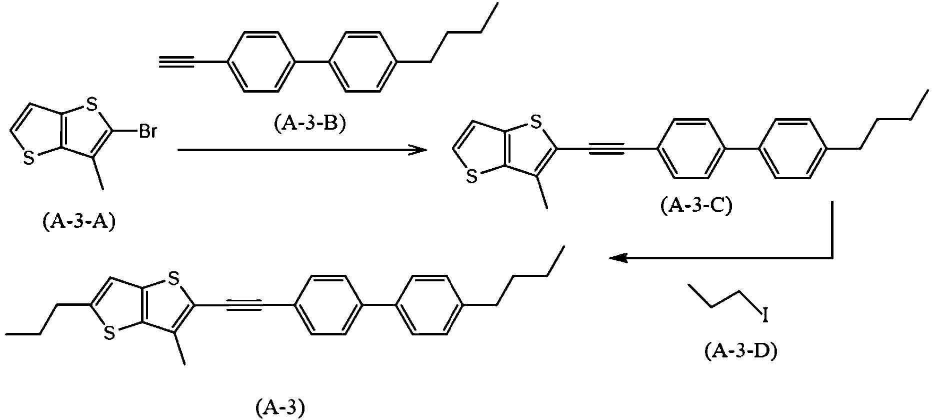

Based on the above, a structural formula of the compound A provided in Embodiment 3 of this application is:

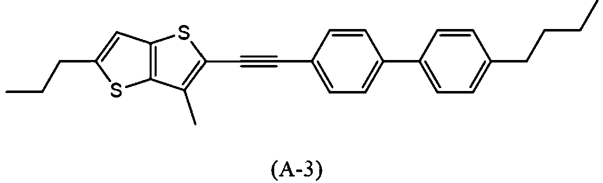

### Embodiment 4

### (1) Preparation of 2-bromo-3-methyl-5-(4-pentylphenyl)thieno[3,2-b]thiophene, "A-4-C" for short.

Specifically, a raw material "A-4-A" (5.5 g, 15.318 mmol), tetrakis(triphenylphosphine)palladium (Pd(PPh₃)₄ for short) (885 mg, 0.76 mmol), and potassium phosphate (K₃PO₄ for short) (6.5 g, 30.6 mmol) were added to a three-necked flask (250 mL). The gas in the three-necked flask was replaced with argon. Water (25 mL) and toluene (100 mL) were added. After the reaction temperature was increased to 75°C, "A-4-B" (3.53 g, 18.38 mmol) dissolved in dimethyl sulfoxide (DMSO for short) was added slowly using an automatic sample injector for reaction for 3 hours. After the reaction was completed, the system was cooled to room temperature, and a product was sequentially filtered with silica gel, washed with water twice, and extracted with ethyl acetate to obtain an organic phase. The organic phase was sequentially washed with a saturated ammonium chloride aqueous solution and a saturated sodium chloride aqueous solution, twice respectively, and dried with anhydrous sodium sulfate. After filtration and spin drying, column chromatography was performed using n-hexane as an eluent for separation and purification to obtain 5.3 g of a white solid substance "A-4-C", with a GC purity of 99%.

### (2) Preparation of 3-methyl-5-(4-pentylphenyl)-2-[(4-propylphenyl)ethynyl]thieno[3,2-b]thiophene, "A-4" for short.

Specifically, a raw material "A-4-C" (5.3 g, 13.99 mmol), [1,1'-bis(diphenylphosphino)ferrocene]dichloropalladium (Pd(dppf)Cl₂ for short) (306 mg, 0.419 mmol), and copper iodide (CuI for short) (159 mg, 0.839 mmol) were added to a three-necked flask (500 mL). The system was kept sealed, and the gas in the three-necked flask was replaced with argon. A raw material "A-4-D" (2.42 g,16.79 mmol), diisopropylamine (20 mL), and DMSO (150 mL) were added. After the reaction temperature was increased to 120°C, the reaction continued for 3 hours. After the reaction was completed, the system was cooled to room temperature, and a product was sequentially filtered with silica gel, washed with water twice, and extracted with ethyl acetate to obtain an organic phase. The organic phase was sequentially washed with a saturated ammonium chloride aqueous solution and a saturated sodium chloride aqueous solution, twice respectively, and dried with anhydrous sodium sulfate. After filtration and spin drying, column chromatography was performed using n-hexane as an eluent for separation and purification to obtain a faint yellow solid. The faint yellow solid was further triturated with methanol to obtain 4.68 g of a substance "A-4", with a GC purity of 99.9%, and GC-MS (EI) m/z=442.

A reaction process as described in (1) and (2) in Embodiment 4 is as set forth in the following formula:

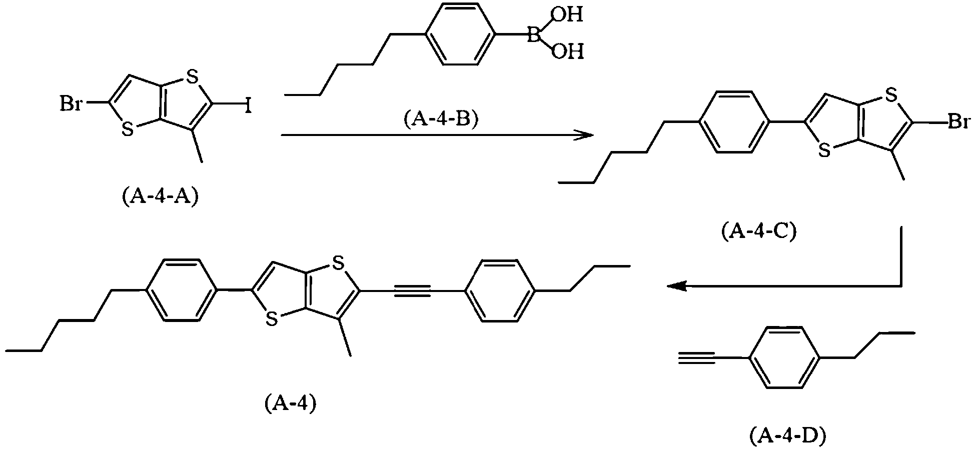

Based on the above, a structural formula of the compound A provided in Embodiment 4 of this application is:

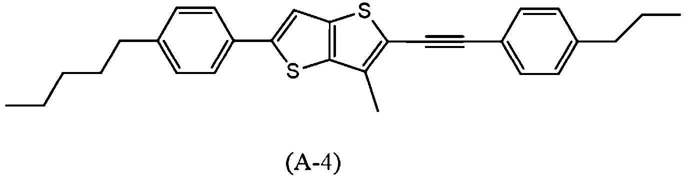

### Embodiment 5

### (1) Preparation of 2-bromo-3-methyl-5-(3,4,5-trifluoro-4λ5,5λ5-cyclohex-1,3,4,5-tetraenyl)thieno[3,2-b]thiophene, "A-5-C" for short.

Specifically, a raw material "A-5-A" (5.5 g, 15.318 mmol), tetrakis(triphenylphosphine)palladium (Pd(PPh₃)₄ for short) (885 mg, 0.76 mmol), and potassium phosphate (K₃PO₄ for short) (6.5 g, 30.6 mmol) were added to a three-necked flask (250 mL). The gas in the three-necked flask was replaced with argon. Water (25 mL) and toluene (100 mL) were added. After the reaction temperature was increased to 75°C, "A-5-B" (3.23 g, 18.38 mmol) dissolved in dimethyl sulfoxide (DMSO for short) was added slowly using an automatic sample injector for reaction for 3 hours. After the reaction was completed, the system was cooled to room temperature, and a product was sequentially filtered with silica gel, washed with water twice, and extracted with ethyl acetate to obtain an organic phase. The organic phase was sequentially washed with a saturated ammonium chloride aqueous solution and a saturated sodium chloride aqueous solution, twice respectively, and dried with anhydrous sodium sulfate. After filtration and spin drying, column chromatography was performed using n-hexane as an eluent for separation and purification to obtain 2.7 g of a yellow solid substance "A-5-C", with a GC purity of 96.5%.

### (2) Preparation of 3-methyl-2-[(4-pentylphenyl)ethynyl]-5-(3,4,5-trifluorophenyl)thieno[3,2-b]thiophene, "A-5" for short.

Specifically, a raw material "A-5-C" (2.7 g, 7.43 mmol), [1,1'-bis(diphenylphosphino)ferrocene]dichloropalladium (Pd(dppf)Cl₂ for short) (163 mg, 0.223 mmol), and copper iodide (CuI for short) (84.9 mg, 0.446 mmol) were added to a three-necked flask (250 mL). The system was kept sealed, and the gas in the three-necked flask was replaced with argon. A raw material "A-5-D" (1.66 g, 9.66 mmol), diisopropylamine (20 mL), and DMSO (80 mL) were added. After the reaction temperature was increased to 120°C, reacted for 3 hours. After the reaction was completed, the system was cooled to room temperature, and a product was sequentially filtered with silica gel, washed with water twice, and extracted with ethyl acetate to obtain an organic phase. The organic phase was sequentially washed with a saturated ammonium chloride aqueous solution and a saturated sodium chloride aqueous solution, twice respectively, and dried with anhydrous sodium sulfate. After filtration and spin drying, column chromatography was performed using n-hexane as an eluent for separation and purification to obtain a faint yellow solid. The faint yellow solid was further triturated with methanol to obtain 2.7 g of a substance "A-5", with a GC purity of 99.9%, and GC-MS (EI) m/z=454.

A reaction process as described in (1) and (2) in Embodiment 5 is as set forth in the following formula:

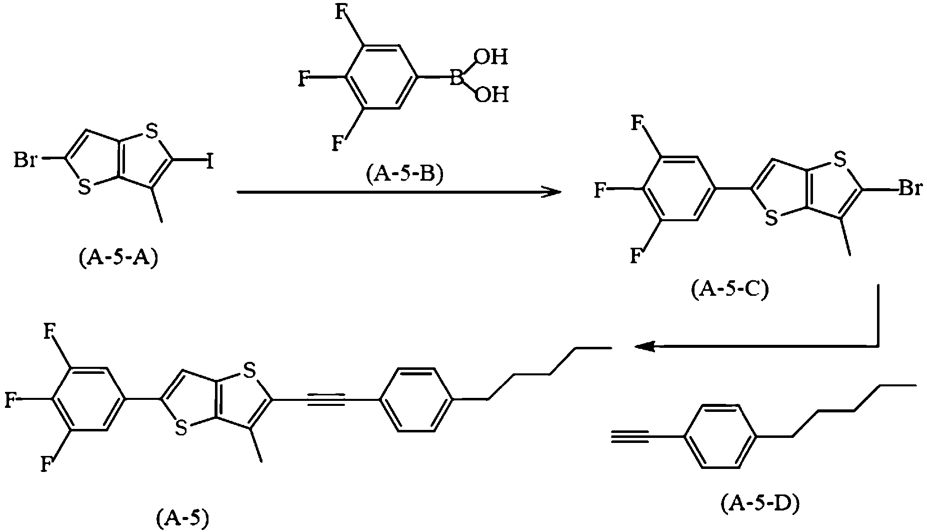

Based on the above, a structural formula of the compound A provided in Embodiment 5 of this application is:

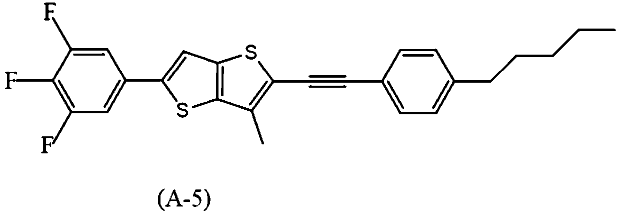

### Embodiment 6

### (1) Preparation of 3-methyl-2-{[4-(4-propylphenyl)phenyl]ethynyl}thieno[3,2-b]thiophene, "A-6-C" for short.

Specifically, a raw material "A-6-B" (15.6 g, 70.8 mmol), [1,1'-bis(diphenylphosphino)ferrocene]dichloropalladium (Pd(dppf)Cl₂ for short) (1.0 g, 1.4 mmol), and copper iodide (CuI for short) (540 mg, 2.8 mmol) were added to a three-necked flask (500 mL). The system was kept sealed, and the gas in the three-necked flask was replaced with argon. A raw material "A-6-A" (16.5 g, 70.8 mmol), diisopropylamine (40 mL), and DMSO (330 mL) were added. After the reaction temperature was increased to 120°C, the reaction continued for 3 hours. After the reaction was completed, the system was cooled to room temperature, and a product was sequentially filtered with silica gel, washed with water twice, and extracted with ethyl acetate to obtain an organic phase. The organic phase was sequentially washed with a saturated ammonium chloride aqueous solution and a saturated sodium chloride aqueous solution, twice respectively, and dried with anhydrous sodium sulfate. After filtration and spin drying, column chromatography was performed using n-hexane as an eluent for separation and purification to obtain a faint yellow solid. The faint yellow solid was further triturated with methanol to obtain 22.35 g of a substance "A-6-C", with a GC purity of 99.6%.

### (2) Preparation of 3-methyl-5-propyl-2-{[4-(4-propylphenyl)phenyl]ethynyl}thieno[3,2-b]thiophene, "A-6" for short.

Specifically, a raw material "A-6-C" (11 g, 29.5 mmol) was added to a three-necked flask (500 mL). The system was kept sealed, and the gas in the three-necked flask was replaced with argon. Tetrahydrofuran (THF) (220 mL) was added and stirred to dissolve until the solution is clear. The system was placed in a -78°C low-temperature reactor and stirred for 0.5 h. Lithium diisopropylamide (LDA for short) (29.5 mL, 59.1 mmol) was dropwise added for reaction for 3 h. "A-6-D" (10.0 g, 59.1 mmol) was dropwise added, and the temperature was increased to room temperature for reaction for 1 h. The reaction was quenched with a saturated ammonium chloride aqueous solution, and a product was sequentially washed with water twice and extracted with ethyl acetate to obtain an organic phase. The organic phase was sequentially washed with a saturated ammonium chloride aqueous solution and a saturated sodium chloride aqueous solution, twice respectively, and dried with anhydrous sodium sulfate. After filtration and spin drying, column chromatography was performed using n-hexane as an eluent for separation and purification to obtain a white solid. The white solid was further recrystallized and triturated with an ethyl acetate/methanol mixed solution for multiple times, to obtain 6.26 g of a substance "A-6", with a GC purity of 99.9%, and GC-MS (EI) m/z=414.

A reaction process as described in (1) and (2) in Embodiment 6 is as set forth in the following formula:

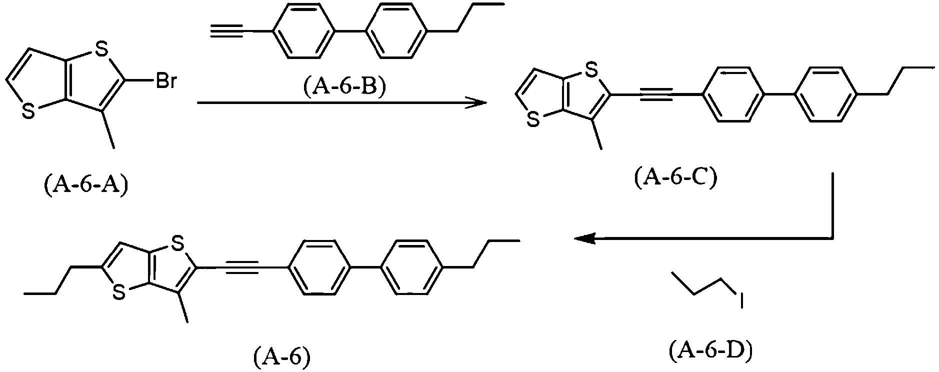

Based on the above, a structural formula of the compound A provided in Embodiment 6 of this application is:

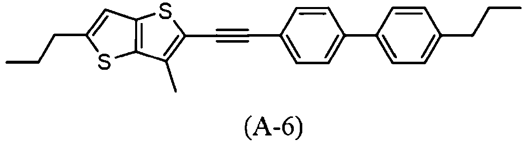

### Embodiment 7

### (1) Preparation of 3-methyl-2-{[4-(4-propylphenyl)phenyl]ethynyl}thieno[3,2-b]thiophene, "A-7-C" for short.

Specifically, a raw material "A-7-B" (15.6 g, 70.8 mmol), [1,1'-bis(diphenylphosphino)ferrocene]dichloropalladium (Pd(dppf)Cl₂ for short) (1.0 g, 1.4 mmol), and copper iodide (CuI for short) (540 mg, 2.8 mmol) were added to a three-necked flask (500 mL). The system was kept sealed, and the gas in the three-necked flask was replaced with argon. A raw material "A-7-A" (16.5 g, 70.8 mmol), diisopropylamine (40 mL), and DMSO (330 mL) were added. After the reaction temperature was increased to 120°C, the reaction continued for 3 hours. After the reaction was completed, the system was cooled to room temperature, and a product was sequentially filtered with silica gel, washed with water twice, and extracted with ethyl acetate to obtain an organic phase. The organic phase was sequentially washed with a saturated ammonium chloride aqueous solution and a saturated sodium chloride aqueous solution, twice respectively, and dried with anhydrous sodium sulfate. After filtration and spin drying, column chromatography was performed using n-hexane as an eluent for separation and purification to obtain a faint yellow solid. The faint yellow solid was further triturated with methanol to obtain 22.35 g of a substance "A-7-C", with a GC purity of 99.6%.

### (2) Preparation of 5-ethyl-3-methyl-2-{[4-(4-propylphenyl)phenyl]ethynyl}thieno[3,2-b]thiophene, "A-7" for short.

Specifically, a raw material "A-7-C" (11 g, 29.5 mmol) was added to a three-necked flask (500 mL). The system was kept sealed, and the gas in the three-necked flask was replaced with argon. Tetrahydrofuran (THF) (220 mL) was added and stirred to dissolve until the solution is clear. The system was placed in a -78°C low-temperature reactor and stirred for 0.5 h. Lithium diisopropylamide (LDA for short) (29.5.7 mL, 59.1 mmol) was dropwise added for reaction for 3 h. "A-7-D" (9.2 g, 59.1 mmol) was dropwise added, and the temperature was increased to room temperature for reaction for 1 h. The reaction was quenched with a saturated ammonium chloride aqueous solution, and a product was sequentially washed with water twice and extracted with ethyl acetate to obtain an organic phase. The organic phase was sequentially washed with a saturated ammonium chloride aqueous solution and a saturated sodium chloride aqueous solution, twice respectively, and dried with anhydrous sodium sulfate. After filtration and spin drying, column chromatography was performed using n-hexane as an eluent for separation and purification to obtain a brown solid. The brown solid was further recrystallized and triturated with an ethyl acetate/methanol mixed solution for multiple times, to obtain 5.97 g of a substance "A-7", with a GC purity of 99.9%, and GC-MS (EI) m/z=400.

A reaction process as described in (1) and (2) in Embodiment 7 is as set forth in the following formula:

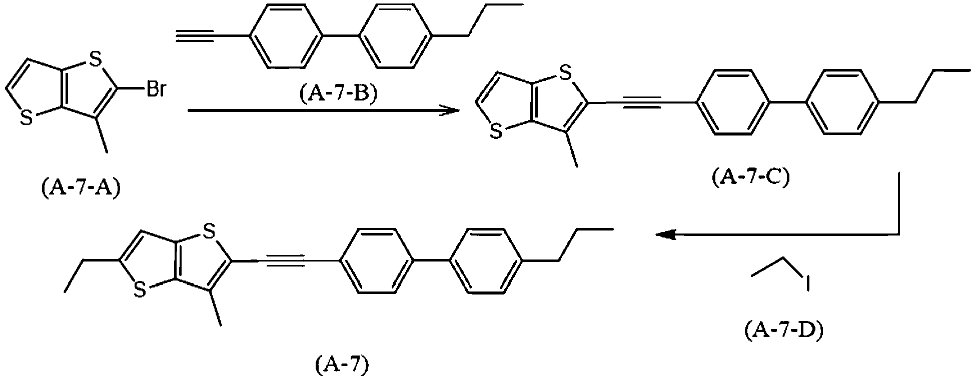

Based on the above, a structural formula of the compound A provided in Embodiment 7 of this application is:

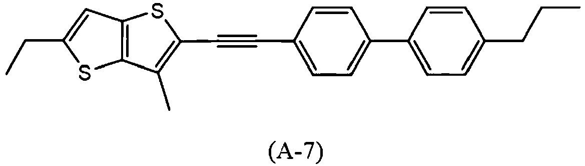

### Embodiment 8

### (1) Preparation of 2-bromo-5-(4-butylphenyl)-3-methylthieno[3,2-b]thiophene, "A-8-C" for short.

Specifically, a raw material "A-8-A" (4.9 g, 13.6 mmol), tetrakis(triphenylphosphine)palladium (Pd(PPh₃)₄ for short) (789 mg, 0.7 mmol), and potassium phosphate (K₃PO₄ for short) (5.8 g, 27.3 mmol) were added to a three-necked flask (250 mL). The gas in the three-necked flask was replaced with argon. Water (25 mL) and toluene (100 mL) were added. After the reaction temperature was increased to 75°C, "A-8-B" (2.9 g, 16.4 mmol) dissolved in dimethyl sulfoxide (DMSO) was added slowly using an automatic sample injector for reaction for 3 hours. After the reaction was completed, the system was cooled to room temperature, and a product was sequentially filtered with silica gel, washed with water twice, and extracted with ethyl acetate to obtain an organic phase. The organic phase was sequentially washed with a saturated ammonium chloride aqueous solution and a saturated sodium chloride aqueous solution, twice respectively, and dried with anhydrous sodium sulfate. After filtration and spin drying, column chromatography was performed using n-hexane as an eluent for separation and purification to obtain 4.15 g of a white solid substance "A-8-C", with a GC purity of 98.5%.

### (2) Preparation of 5-(4-butylphenyl)-3-methyl-2-[(4-propylphenyl)ethynyl]thieno[3,2-b]thiophene, "A-8" for short.

Specifically, a raw material "A-8-C" (4.15 g, 11.4 mmol), [1,1'-bis(diphenylphosphino)ferrocene]dichloropalladium (Pd(dppf)Cl₂ for short) (249 mg, 0.3 mmol), and copper iodide (CuI for short) (131 mg, 0.7 mmol) were added to a three-necked flask (250 mL). The system was kept sealed, and the gas in the three-necked flask was replaced with argon. A raw material "A-8-D" (1.97 g, 13.6 mmol), diisopropylamine (5 mL), and DMSO (100 mL) were added. After the reaction temperature was increased to 120°C, the reaction continued for 3 hours. After the reaction was completed, the system was cooled to room temperature, and a product was sequentially filtered with silica gel, washed with water twice, and extracted with ethyl acetate to obtain an organic phase. The organic phase was sequentially washed with a saturated ammonium chloride aqueous solution and a saturated sodium chloride aqueous solution, twice respectively, and dried with anhydrous sodium sulfate. After filtration and spin drying, column chromatography was performed using n-hexane as an eluent for separation and purification to obtain a faint yellow solid. The faint yellow solid was further triturated with methanol to obtain 3.92 g of a substance "A-8", with a GC purity of 99.9%, and GC-MS (EI) m/z=428.

A reaction process as described in (1) and (2) in Embodiment 8 is as set forth in the following formula:

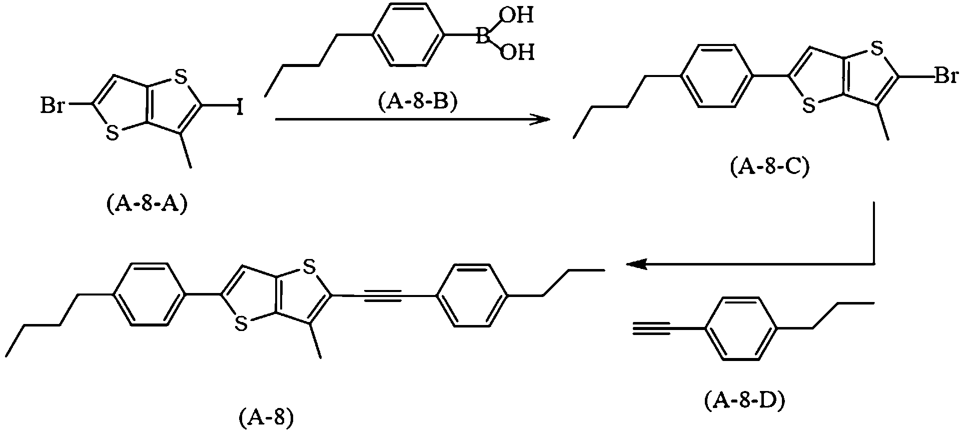

Based on the above, a structural formula of the compound A provided in Embodiment 8 of this application is:

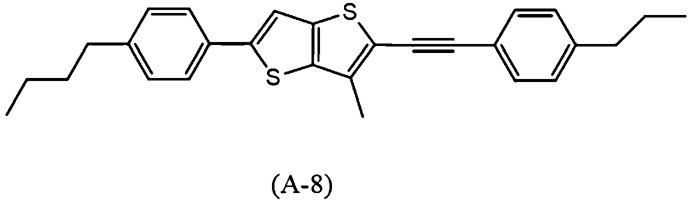

### Embodiment 9

### (1) Preparation of 2-bromo-3-methyl-5-(4-propylphenyl)thieno[3,2-b]thiophene, "A-9-C" for short.

Specifically, a raw material "A-9-A" (14 g, 38.99 mmol), tetrakis(triphenylphosphine)palladium (Pd(PPh₃)₄ for short) (2.25 g, 1.94 mmol), and potassium phosphate (K₃PO₄ for short) (16.5 g, 70.99 mmol) were added to a three-necked flask (500 mL). The gas in the three-necked flask was replaced with argon. Water (40 mL) and toluene (260 mL) were added. After the reaction temperature was increased to 75°C, "A-9-B" (7.67 g, 46.79 mmol) dissolved in dimethyl sulfoxide (DMSO for short) was added slowly using an automatic sample injector for reaction for 3 hours. After the reaction was completed, the system was cooled to room temperature, and a product was sequentially filtered with silica gel, washed with water twice, and extracted with ethyl acetate to obtain an organic phase. The organic phase was sequentially washed with a saturated ammonium chloride aqueous solution and a saturated sodium chloride aqueous solution, twice respectively, and dried with anhydrous sodium sulfate. After filtration and spin drying, column chromatography was performed using n-hexane as an eluent for separation and purification to obtain 11.89 g of white solid "A-9-C", with a GC purity of 99.2%.

### (2) Preparation of 3-methyl-5-(4-propylphenyl)-2-[(4-propylphenyl)ethynyl]thieno[3,2-b]thiophene, "A-9" for short.

Specifically, a raw material "A-9-C" (5.9 g, 16.79 mmol), [1,1'-bis(diphenylphosphino)ferrocene]dichloropalladium (Pd(dppf)Cl₂ for short) (368 mg, 0.503 mmol), and copper iodide (CuI for short) (191 mg, 1.007 mmol) were added to a three-necked flask (250 mL). The system was kept sealed, and the gas in the three-necked flask was replaced with argon. A raw material "A-9-D" (3.14 g, 21.83 mmol), diisopropylamine (20 mL), and DMSO (160 mL) were added. After the reaction temperature was increased to 120°C, reacted for 3 hours. After the reaction was completed, the system was cooled to room temperature, and a product was sequentially filtered with silica gel, washed with water twice, and extracted with ethyl acetate to obtain an organic phase. The organic phase was sequentially washed with a saturated ammonium chloride aqueous solution and a saturated sodium chloride aqueous solution, twice respectively, and dried with anhydrous sodium sulfate. After filtration and spin drying, column chromatography was performed using n-hexane as an eluent for separation and purification to obtain a yellow solid. The yellow solid was further triturated with methanol to obtain 6.36 g of a substance "A-9-C", with a GC purity of 99.9%, and GC-MS (EI) m/z=414.

A reaction process as described in (1) and (2) in Embodiment 9 is as set forth in the following formula:

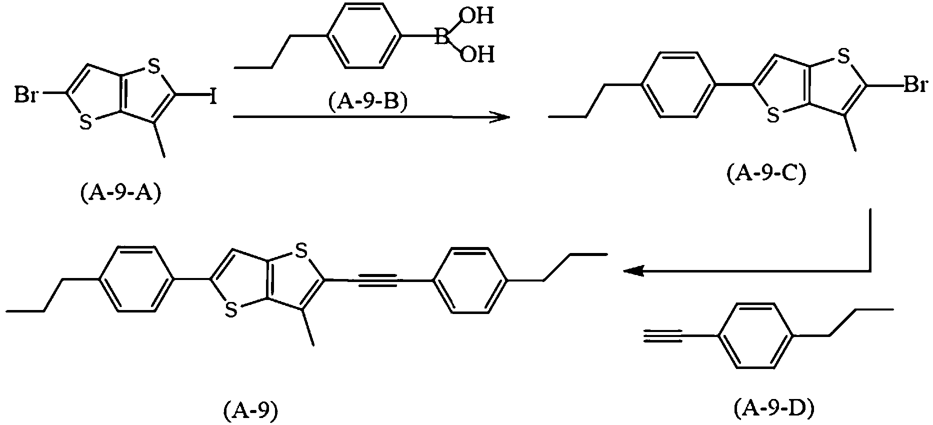

Based on the above, a structural formula of the compound A provided in Embodiment 9 of this application is:

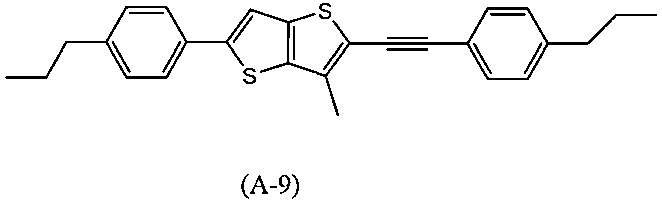

### Embodiment 10

### (1) 2-bromo-3-methyl-5-(4-propylphenyl)thieno[3,2-b]thiophene, "A-10-C" for short.

Specifically, a raw material "A-10-A" (14 g, 38.99 mmol), tetrakis(triphenylphosphine)palladium (Pd(PPh₃)₄ for short) (2.25 g, 1.94 mmol), and potassium phosphate (K₃PO₄ for short)(16.5 g, 70.99 mmol) were added to a 500 mL three-necked flask. The gas in the three-necked flask was replaced with argon. Water (40 mL) and toluene (260 mL) were added. After the reaction temperature was increased to 75°C, "A-10-B" (7.67 g, 46.79 mmol) dissolved in dimethyl sulfoxide (DMSO for short) was added slowly using an automatic sample injector for reaction for 3 hours. After the reaction was completed, the system was cooled to room temperature, and a product was sequentially filtered with silica gel, washed with water twice, and extracted with ethyl acetate to obtain an organic phase. The organic phase was sequentially washed with a saturated ammonium chloride aqueous solution and a saturated sodium chloride aqueous solution, twice respectively, and dried with anhydrous sodium sulfate. After filtration and spin drying, column chromatography was performed using n-hexane as an eluent for separation and purification to obtain 11.89 g of a white solid "A-10-C", with a GC purity of 99.2%.

### (2) Preparation of 2-[(4-ethylphenyl)ethynyl]-3-methyl-5-(4-propylphenyl)thieno[3,2-b]thiophene, "A-10" for short.

Specifically, a raw material "A-10-C" (5.9 g, 16.79 mmol), [1,1'-bis(diphenylphosphino)ferrocene]dichloropalladium (Pd(dppf)Cl₂ for short) (368 mg, 0.503 mmol), and copper iodide (CuI for short) (191 mg, 1.007 mmol) were added to a 500 mL three-necked flask. The system was kept sealed, and the gas in the three-necked flask was replaced with argon. A raw material "A-10-D" (2.84 g, 21.83 mmol), diisopropylamine (20 mL), and DMSO (160 mL) were added. After the reaction temperature was increased to 120°C, the reaction continued for 3 hours. After the reaction was completed, the system was cooled to room temperature, and a product was sequentially filtered with silica gel, washed with water twice, and extracted with ethyl acetate to obtain an organic phase. The organic phase was sequentially washed with a saturated ammonium chloride aqueous solution and a saturated sodium chloride aqueous solution, twice respectively, and dried with anhydrous sodium sulfate. After filtration and spin drying, column chromatography was performed using n-hexane as an eluent for separation and purification to obtain a yellow solid. The yellow solid was further triturated with methanol to obtain 5.8 g of a substance "A-10", with a GC purity of 99.9%, and GC-MS (EI) m/z=400.

A reaction process as described in (1) and (2) in Embodiment 10 is as set forth in the following formula:

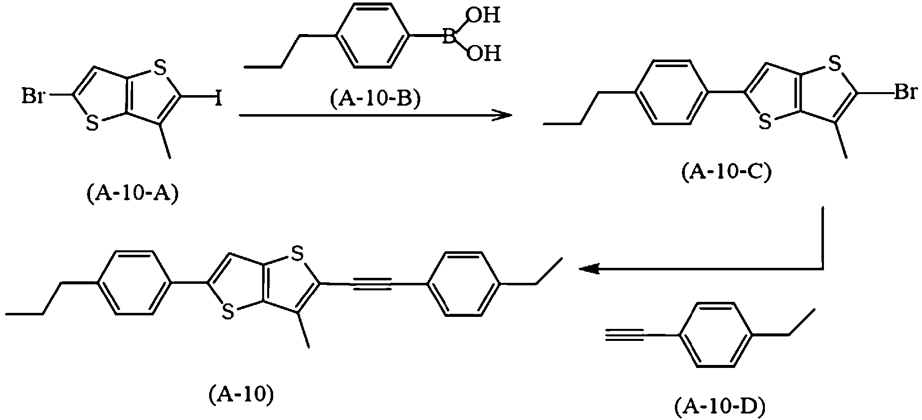

Based on the above, a structural formula of the compound A provided in Embodiment 10 of this application is:

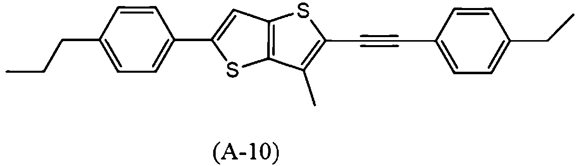

The 10 kinds of compound A provided by the foregoing embodiments are merely used as examples, and do not constitute a limitation on embodiments of this application. The compound A provided by embodiments of this application may be of any structure that meets the general formula as set forth in formula (I), which will not be listed one by one herein.

The birefringence Δn, the dielectric anisotropy Δε, and the clearing point CP of each of the compounds (A-1, A-2, A-3, and A-5) provided by embodiments of this application are tested.

Δn (589 nm, 25°C) = nₑ (589 nm, 25°C) -nₒ (589 nm, 25°C). The values of nₑ (589 nm, 25°C) and nₒ (589 nm, 25°C) are measured using an ATAGO-DR-M4 Abbe refractometer.

Δε (1 kHz, 25°C) = ε_{//}(1 kHz, 25°C) -ε_{⊥} (1 kHz, 25°C). ε_{//} represents a dielectric constant in a parallel direction of a liquid crystal, and ε_{⊥} represents a dielectric constant in a vertical direction of a liquid crystal. Both ε_{//} (1 kHz, 25°C) and ε_{⊥} (1 kHz, 25°C) are measured using an ALCT-IV1 liquid crystal comprehensive parameter tester.

CP represents the clearing point of a liquid crystal, and is measured by a METTLER DSC3 differential scanning calorimeter with a temperature rising rate of 10 °C/min in a nitrogen atmosphere, where CP is measured in °C and represented by T_{NI} (°C).

Test results are shown in Table 1.

**Table 1**

| Compound A | Δn | Δε | T_{NI} (°C) |
|---|---|---|---|
| A-1 | 0.48 | 3.6 | 256 |
| A-2 | 0.45 | 3.1 | 250 |
| A-3 | 0.45 | 3.1 | 254 |
| A-5 | 0.40 | 14 | 165 |

A compound A provided by a comparative example of this application may have the following structure B-1.

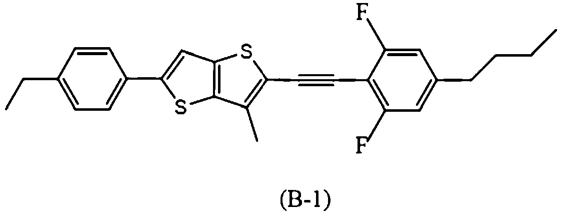

The aforementioned compound having the structural formula A-1 was used as a liquid crystal monomer, and dissolved in a mother liquid crystal at a mass percentage of 10%, to obtain a liquid crystal composition sample 1. The compound having the structural formula B-1 provided by the aforementioned comparative example was used as a liquid crystal monomer, and dissolved in a mother liquid crystal at a mass percentage of 10%, to obtain a liquid crystal composition sample 2. The mother liquid crystal was prepared by mixing monomers such as phenylcyclohexane, biphenylcyclohexane and terphenyl.

A voltage holding ratio test was performed on the liquid crystal composition sample 1 and the liquid crystal composition sample 2 by the following method, and test results are shown in Table 2.

First, the liquid crystal composition sample 1 was poured into two TN-type liquid crystal boxes with a box thickness of 7 µm and sealed, to obtain a liquid crystal box sample 1 and a liquid crystal box sample 2. The liquid crystal composition sample 2 was poured into two TN-type liquid crystal boxes with a box thickness of 7 µm and sealed, to obtain a liquid crystal box sample 3 and a liquid crystal box sample 4.

Then, the liquid crystal box sample 1 was placed in an ultraviolet test chamber, and ultraviolet aging treatment was performed on the liquid crystal composition sample 1 by using a 365 nm light source and accumulating a set irradiation dose. The liquid crystal box sample 2 was placed in a high-temperature incubator with adjustable high temperature and low temperature, and high-temperature aging treatment was performed on the liquid crystal composition sample 1 by setting the temperature to 100°C, and taking out the sample after a preset baking time. The liquid crystal box sample 3 was placed in an ultraviolet test chamber, and ultraviolet aging treatment was performed on the liquid crystal composition sample 2 by using a 365 nm light source and accumulating a set irradiation dose. The liquid crystal box sample 4 was placed in a high-temperature incubator with adjustable high temperature and low temperature, and high-temperature aging treatment was performed on the liquid crystal composition sample 2 by setting the temperature to 100°C, and taking out the sample after a preset baking time.

Finally, before and after the aging, the voltage holding ratio (V_{HR}) of each of the liquid crystal box sample 1, the liquid crystal box sample 2, the liquid crystal box sample 3, and the liquid crystal box sample 4 was tested using the ALCT-IV1 liquid crystal comprehensive parameter tester under a frequency of 60 Hz, a voltage of 5 V, and a temperature of 25°C.

**Table 2**

| Monomer | Ultraviolet (365 nm) aging test | | | High temperature (100°C) aging test | | |
|---|---|---|---|---|---|---|
| | V_{HR} (initial) | V_{HR} (40J) | ΔV_{HR} | V_{HR} (initial) | V_{HR} (500 h) | ΔV_{HR} |
| A-1 | 99.18 | 82.04 | 17.15 | 99.46 | 71.22 | 24.15 |
| B-1 | 98.78 | 62.21 | 36.57 | 99.86 | 59.99 | 37.08 |

As can be seen from the above table, compared to the compound having the structure B-1 provided by the comparative example, the voltage holding ratio of the compound having the structure A-1 provided by embodiments of this application is reduced by a smaller amplitude. Therefore, the performance of a device including the liquid crystal compound material as described in the present invention will also be more stable.

The foregoing descriptions are merely specific implementations of the present invention, and are not intended to limit the protection scope of the present invention. Any variation or replacement readily figured out by a person skilled in the art within the technical scope disclosed in the present invention shall fall within the protection scope of the present invention. Therefore, the protection scope of the present invention shall be subject to the protection scope of the claims.

## Claims

1. A compound, having a structure as set forth in formula (I), 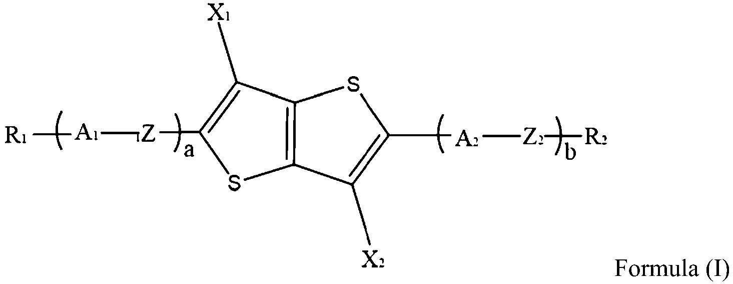
wherein X₁ and X₂ are each independently selected from hydrogen, deuterium, tritium, at least one halogen and at least one hydrocarbyl group, and at least one of X₁ and X₂ is not hydrogen;
R₁ is selected from at least one hydrocarbyl group;
R₂ is selected from at least one polar group and at least one hydrocarbyl group;
A₁ and A₂ are each independently selected from an arylene group, a heteroarylene group, a cyclic hydrocarbylene group, and a heterocyclic hydrocarbylene group;
Z₁ and Z₂ are each independently a linking group or a chemical bond; and
a and b are integers.

2. The compound according to claim 1, wherein when X₁ is selected from at least one hydrocarbyl group, X₁ is of a straight chain structure, or has a branched chain structure; and/or when X₂ is selected from at least one hydrocarbyl group, X₂ is of a straight chain structure, or has a branched chain structure.

3. The compound according to claim 1 or 2, wherein when X₁ is selected from at least one hydrocarbyl group, at least one hydrogen atom on X₁ is substituted with a fluorine atom; and/or when X₂ is selected from at least one hydrocarbyl group, at least one hydrogen atom on X₂ is substituted with a fluorine atom.

4. The compound according to any one of claims 1 to 3, wherein when X₁ is selected from at least one hydrocarbyl group, X₁ comprises 1 to 8 carbon atoms; and/or when X₂ is selected from at least one hydrocarbyl group, X₂ comprises 1 to 8 carbon atoms.

5. The compound according to any one of claims 1 to 4, wherein X₁ and X₂ are each independently an alkyl group, an alkoxy group, an alkenyl group, an alkenyloxy group, a cycloalkyl group, or a cycloalkenyl group.

6. The compound according to claim 5, wherein
the alkyl group comprises 1 to 8 carbon atoms;
the alkoxy group comprises 1 to 7 carbon atoms;
the alkenyl group comprises 2 to 8 carbon atoms;
the alkenyloxy group comprises 2 to 7 carbon atoms;
the cycloalkyl group comprises 3 to 8 carbon atoms; and
the cycloalkenyl group comprises 3 to 8 carbon atoms.

7. The compound according to claim 1, wherein at least one of X₁ and X₂ is a methyl group.

8. The compound according to claim 1, wherein R₁ and R₂ are each independently selected from an alkyl group, an alkoxy group, an alkenyl group, an alkenyloxy group, an alkynyl group and an alkynyloxy group that are of a straight chain structure or have a branched chain structure;
the alkyl group, the alkoxy group, the alkenyl group, the alkenyloxy group, the alkynyl group and the alkynyloxy group contain one or more of oxygen, sulfur and nitrogen;
any hydrogen atom on the alkyl group, the alkoxy group, the alkenyl group, the alkenyloxy group, the alkynyl group and the alkynyloxy group is optionally substituted with a deuterium atom, a tritium atom or a halogen atom; and
any two non-adjacent carbon atoms in the alkyl group, the alkoxy group, the alkenyl group, the alkenyloxy group, the alkynyl group and the alkynyloxy group bond together to form a cyclic ring structure.

9. The compound according to claim 8, wherein
the alkyl group and the alkoxy group comprise 1 to 20 carbon atoms; and
the alkenyl group, the alkenyloxy group, the alkynyl group and the alkynyloxy group comprise 2 to 20 carbon atoms.

10. The compound according to any one of claims 1 to 9, wherein the at least one polar group comprises F, Cl, Br, CN, NO₂, SCN, NCS, NCSe, OCF₃ and SF₅.

11. The compound according to any one of claims 1 to 10, wherein at least one hydrogen atom on A₁ and/or A₂ is substituted with X₃, and X₃ is selected from a deuterium atom, a tritium atom, a halogen atom, a straight-chain or branched-chain alkyl group with 1 to 8 carbon atoms, a straight-chain or branched-chain alkoxy group with 1 to 7 carbon atoms, a straight-chain or branched-chain alkenyl group with 2 to 8 carbon atoms, a straight-chain or branched-chain alkenyloxy group with 2 to 7 carbon atoms, a cycloalkyl group with 3 to 8 carbon atoms, and a cycloalkenyl group with 3 to 8 carbon atoms; and
any hydrogen atom on the alkyl group, the alkoxy group, the alkenyl group, the alkenyloxy group and the cycloalkenyl group is substituted with a fluorine atom.

12. The compound according to claim 1 or 11, wherein
the arylene group comprises 6 to 60 carbon atoms;
the heteroarylene group comprises 2 to 60 carbon atoms;
the cyclic hydrocarbylene group comprises 3 to 60 carbon atoms; and
the heterocyclic hydrocarbylene group comprises 1 to 60 carbon atoms.

13. The compound according to any one of claims 1 to 12, wherein when a plurality of A₁s are present in formula (I), the plurality of A₁s are the same or different; and when a plurality of A₂s are present in formula (I), the plurality of A₂s are the same or different.

14. The compound according to any one of claims 1 to 13, wherein Z₁ and Z₂ are each independently selected from -CH=CH-, -CF=CF-, -CH=CF-, -CF=CH-, -C=C-, -C≡C-C≡C-, -CO-O-, -O-CO, -CF₂O-, -OCF₂-, -CH₂CH₂-, -N=N-, -CH=N-, -N=CH-, -CH₂O-, -OCH₂-, -CF₂CF₂-, -CF₂CH₂-, and -CH₂CF₂-.

15. The compound according to any one of claims 1 to 14, wherein a is selected from 0, 1, 2 and 3; and b is selected from 0, 1, 2 and 3.

16. A liquid crystal composition, comprising the compound according to any one of claims 1 to 15.

17. Uses of the compound according to any one of claims 1 to 15 or the composition according to claim 16 in a display device, an optical communication device, a wireless communication device, and a lens component.
